# EUROPEAN PATENT SPECIFICATION

(11) **EP 1 701 973 B1**
(45) Date of publication and mention of the grant of the patent: **15.09.2010**
(21) Application number: 04805051.2
(22) Date of filing: 29.12.2004
(51) Int. Cl.: C07K 5/02, A61K 38/06

(54) **GLUTATHIONE DERIVATIVES AND THEIR USES FOR THE TREATMENT OF VIRAL DISEASES**
GLUTATHIONDERIVATE UND DEREN VERWENDUNG ZUR BEHANDLUNG VON VIRALEN ERKRANKUNGEN
DERIVES DE GLUTATHION ET UTILISATIONS DE CES DERNIERS POUR LE TRAITEMENT DES MALADIES VIRALES

(30) Priority: 30.12.2003 IT TO20031048
(43) Date of publication of application: 20.09.2006
(73) Proprietor: FIRST - SOCIETA' A RESPONSABILITA' LIMITATA CON SOCIO UNICO, Roma (IT)
(72) Inventor: Benatti, Umberto, 16125 Genova (IT); Brandi, Giorgio, 61033 Fermignano (IT); Garaci, Enrico, 00198 Roma (IT); Magnani, Mauro, 61029 Urbino (IT); Millo, Enrico, 16131 Genova (IT); Palamara, Anna Teresa, 00196 Roma (IT); Rossi, Luigia, 61029 Urbino (IT)
(74) Representative: Jorio, Paolo
(86) International application number: PCT/EP2004/053726
(87) International publication number: WO 2005/063795

(56) References cited:
- WO-A-98/09986
- US-A- 5 464 825
- DATABASE WPI Section Ch, Week 198807 Derwent Publications Ltd., London, GB; Class B04, AN 1988-045801 XP002322756 & JP 63 002922 A (YAMANOUCHI PHARM CO LTD) 7 January 1988 (1988-01-07)
- DATABASE WPI Section Ch, Week 198239 Derwent Publications Ltd., London, GB; Class D21, AN 1982-82123E XP002322757 & JP 57 134410 A (POLA KASEI KOGYO KK) 19 August 1982 (1982-08-19)
- PALAMARA ANNA TERESA ET AL: "Evidence for antiviral activity of glutathione: In vitro inhibition of herpes simplex virus type 1 replication" ANTIVIRAL RESEARCH, vol. 27, no. 3, 1995, pages 237-253, XP002322755 ISSN: 0166-3542 cited in the application

## Description

### TECHNICAL FIELD

The present invention relates to glutathione derivatives of formula I: 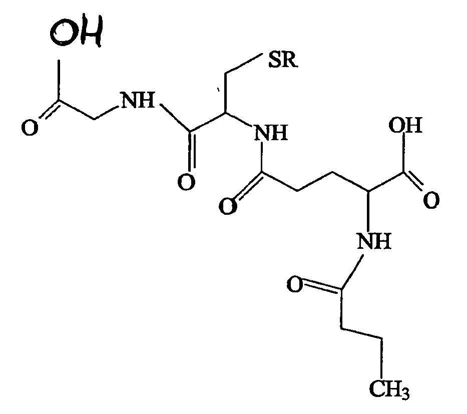 and to their use as antiviral drugs, in particular for the treatment of Paramyxovirus, Orthomyxovirus, Herpes virus and HIV.

Glutathione (also known by the abbreviation GSH) is a tripeptide (γ-glutamylcysteinylglycine) containing cysteine which is found in the eukaryotic cells in millimolar concentrations and has numerous functions in cellular physiology: it protects the cells from oxidative stress, maintaining the intracellular redox state in reducing conditions, through metabolic interconversion, in its oxidized form of disulphide.

### BACKGROUND ART

It is known and reported in numerous articles that during viral infections there is a progressive reduction in GSH with consequent alteration of intracellular redox balance.

Unfortunately, GSH in vivo is oxidized rapidly, particularly in the presence of viral infections, a condition in which the redox state of the cells is unbalanced. Oxidized GSH is in turn reduced by the cellular glutathione reductase or eliminated from the cell through an ATP-dependent mechanism. Reduction of glutathione through cellular enzymes depends on the availability of reducing equivalents in the cell (NADH, NADPH) which are already depleted in the case of pathogen infection.

Recent data report, for example, that during infections in vitro with the Sendai parainfluenza 1 virus (SV), the Herpes Simplex-1 virus (HSV-1) and the Human Immunodeficiency Virus (HIV) a progressive decrease in GSH levels occurs. Many in vivo studies also report the existence of an unbalance of the redox state in the cells and body fluids of patients affected by HIV and Hepatitis C. Moreover, during experimental viral infections with the influenza virus a decrease in the anti-oxidant defenses and simultaneous increase in lipid oxidation products in the lungs and livers of animals sacrificed on the seventh day after infection have been described.

Many data suggest that an unbalance of the intracellular redox state is a key event in the replicative cycle of viruses, as it causes both an increase in replication and activation of nuclear transcription factors.

It is also known that the administration of reduced glutathione to infected cells prevents the decrease in intracellular GSH and inhibits viral replication in SV, HSV-1 and HIV infections, as reported, for example, in the article by Palamara, A. T., Perno, C. F., Ciriolo, M. R., Dini, L., Balestra, E., D'Agostini, C. et al. (1995) "Evidence for antiviral activity of glutathione: in vitro inhibition of herpes simplex virus type 1 replication" in Antiviral Research 27, 237-253, and in the article by Garaci, E., Palamara, A.T., Di Francesco, P., Favalli, C., Ciriolo, M. R. & Rotilio, G. (1992), "Glutathione inhibits replication and expression of viral proteins in cultured cells infected with Sendai virus" in Biochemical and biophysical research communications 188, 1090-1096.

In experimental models the antiviral activity of GSH seems to be correlated to an inhibition of the post-transcriptional stages of virus replication, probably preventing correct folding and maturation of specific proteins.

The efficacy of anti-oxidant substances in viral infections has been proven by in vivo studies, in particular in the article by Palamara, A. T., Garaci, E., Rotilio, G., Ciriolo, M. R., Casabianca, A., Fraternale, A. et al. (1996c) "Inhibition of murine AIDS by reduced glutathione" in AIDS research and human retroviruses 12, 1373-1381.

Administration of high doses of GSH reduces the viral infection and inhibits advance of the disease, even, for example, in a murine model of AIDS.

However, one problem which occurs in the use and administration of GSH, which is the problem addressed by the present invention, is the fact that, although antiviral activity of the antioxidant substances has been clearly proven, it has also been proven that GSH is not transported as such in the majority of cells or tissues.

### DISCLOSURE OF INVENTION

For this reason it would be desirable to obtain molecules which maintain the advantageous characteristics of GSH, while at the same time allowing the problem of transport to be solved, and which therefore facilitate crossing the cellular membrane of many types of cells.

According to the present invention, this problem is solved by glutathione derivatives of formula I.

### BRIEF DESCRIPTION OF THE DRAWINGS

The invention will now be described also with reference to the accompanying figures, in which:
- Figure 1 shows the structural formula of a glutathione derivative according to the present invention, that is, n-butanoyl γ-glutamylcysteinylglycine (also known by the abbreviation GSH-C4), together with its mass spectrometry analysis after HPLC purification. The analysis, conducted in negative ion mode, highlighted the presence of a single mass peak 376.7 corresponding to the monocharged molecule of interest ([M-H]⁻).
- Figure 2 shows the effect of GSH-C4 on Sendai virus replication.
- Figure 3 shows the effects of GSH-C4 on HSV-1 replication.

According to the present invention, with the glutathione derivatives (GSH) of formula I a strong antiviral activity can be obtained in vitro, both against RNA viruses (parainfluenza-1, Sendai), and DNA viruses (Herpes Simplex, HSV-1), crossing the cellular membrane both of MDCK cells and of Vero cells, and without causing toxic effects on uninfected cells. GSH can be considered an antimicrobial agent which exercises its activity through different mechanisms depending on the host-pathogen system in question and on the concentration used.

The derivatives of the present invention are obtained by condensation of a carboxylic acid on the group µ-NH₂ of the glutamic acid.

The compounds were synthesized in the laboratory using conventional solid phase peptide synthesis methods.

It was found that the butanoyl-glutathione according to the invention, indicated hereunder for convenience also with the abbreviation GSH-C4, acts with a different mechanism to the one shown for glutathione (GSH). In fact, besides significantly inhibiting HSV-1 replication, it is capable of preventing the cytopathic effects induced by the virus in Vero cells, and it also inhibits Sendai parainfluenza virus replication in MDCK cells, probably by interfering with essential cellular factors during the viral infection.

One of the advantages of the invention consists in the fact that GSH-C4 or a derivative thereof of formula I can preferably be used as a drug soluble in water, but also in cream or lotion form for the treatment of the Herpes Simplex 1 and 2 pathologies by topical administration.

Butanoyl glutathione of formula I can be considered an interesting antimicrobial agent against various pathogenic agents, as, according to one characteristic of the invention, it reduces both viral infectivity in the first stages of the disease, and viral production at a more advanced stage, an advantageous characteristic which can, at the present time, be considered unique.

### BEST MODE FOR CARRYING OUT THE INVENTION

The invention is now described with reference to specific examples of embodiment and of tests of the derivative according to the invention, without the invention being intended as limited to said examples.

### Example 1

### Synthesis of glutathione derivatives (GSH-C2, GSH-C4, GSH-C6, GSH-C8, GSH-C12)

The compounds were synthesized in the laboratory using conventional solid phase peptidic synthesis methods, in particular using the Fmoc (9-fluorenylmethoxycarbonyl) technique, suitably modified. It was then found that butanoyl glutathione GSH-C4 alone was able to solve the technical problem addressed by the present invention and therefore the subsequent experiments were performed only on this derivative.

All laboratory syntheses were conducted with the manual technique using a suitable reaction container containing a polystyrene resin (Wang-Gly-Fmoc resin produced by Novabiochem AG, Laufelfingen, Switzerland) functionalized with a glycine which has the N-terminal group protected by the Fmoc group.

The standard synthesis cycle comprised the phase of pretreatment of the resin by suspension in dichloromethane (Biosolve LTD, Netherlands) for one night, after which the Fmoc protection group was removed with piperidine (Fluka Chemie AG, Buchs, Switzerland) in N,N-dimethylformamide (DMF) (Biosolve LTD, Netherlands) for 20 minutes.

Simultaneously, 5 equivalents (eq.) of the appropriate Fmoc amino acid (Advanced Biotech Italia, Italy) were preactivated with 4.5 eq. of O-benzotriazol-1-yl)1,1,3,3-tetramethyluronium hexafluorophosphate (HATU) (Advanced Biotech Italy), 5 eq. of N,N diisopropylethylamine, at the final concentration of 0.2 M in anhydrous N-methylpyrrolidone (Biosolve LTD, Netherlands). This solution was made to react with the resin neutralized in situ for approximately 1 hour at 40°C.

Subsequently, a reaction is advisably performed with a 5% acetic anhydride solution (Fluka Chemie AG, Buchs, Switzerland) in DMF to avoid undesired reactions of any amine groups that remain free.

### Example 2

The butanoyl glutathione derivative (GSH-C4) was prepared for treatment of the resin containing the peptidic portion with n-butanoic acid (Fluka Chemie AG, Buchs, Switzerland) previously activated as already described for the amino acids. The derivatives ethanoyl, exanoyl, octanoyl and dodecanoyl (GSH-C2, GSH-C6, GSH-C8 and GSH-C12) were prepared with similar methods, respectively using acetic anhydride and hexanoic, octanoic and dodecanoic acids (Fluka Chemie AG, Buchs, Switzerland) in place of butanoic acid.

Detachment of the synthesized compound from the resin and simultaneous removal of the side chain protector groups was conducted using a solution composed of trifluoroacetic acid (TFA) (Biosolve LTD, Netherlands), ethandithiol (FlukaChemie AG, Buchs, Switzerland), water and triisopropylsilane (FlukaChemie AG, Buchs, Switzerland) in proportion 92.5:2.5:2.5:1 v/v for a time of 2 hours at room temperature. The acid solution was vacuum concentrated to approximately 1 ml of final volume and the final product precipitated with cold diethyl ether and subsequently washed with the same solvent.

All the molecules were purified by reverse phase liquid chromatography (RP-HPLC) using a Waters C18 µBondapack column, in which the solvent A was composed of 0.1% TFA in water and the solvent B of the same percentage of acid in acetonitrile. Elution of the compounds took place with a gradient that started at 100% of solvent A for 5 minutes, increased linearly up to 60% of solvent B in 30 minutes and finally terminated with 100% of B in 5 minutes.

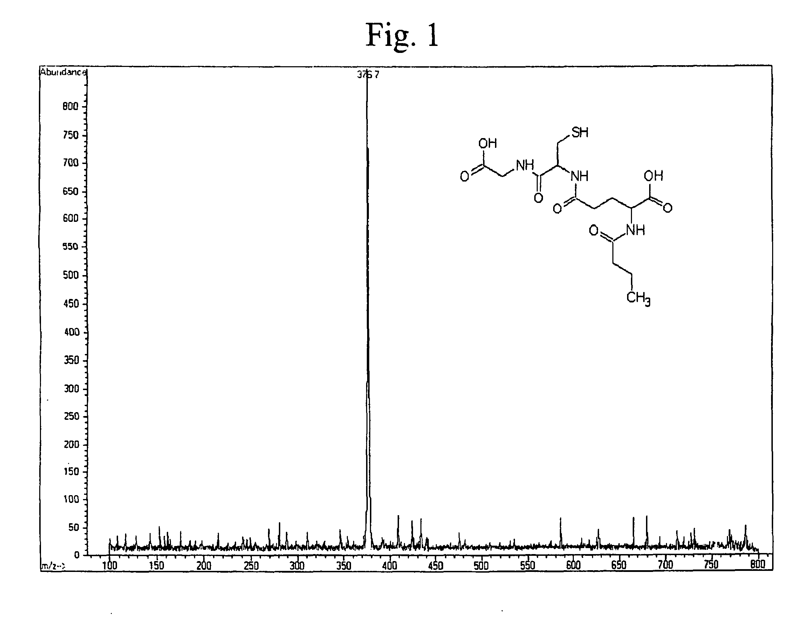

The fractions containing the molecules of interest were collected, vacuum concentrated and then lyophilized. The molecular weights of the glutathione derivatives were confirmed by mass spectrometry analysis. The mass spectra of each compound were acquired using an HP Engine 5989-A spectrometer with a single quadruple equipped with an electrospray source in negative ion mode. All products were obtained with a final yield varying from 75-78% and a purity of 95% after HPLC analysis. An example of the mass spectra obtained is shown in Figure 1.

### Example 3

All the compounds obtained (GSH-C2, GSH-C4, GSH-C6, GSH-C8, GSH-C12) were assayed on confluent monolayers of uninfected Madin Darby canine kidney cells (MDKC) for studies on the toxicity on the cells. The toxicity was measured on the basis of microscopic examinations of cellular morphology, of measurement of cellular vitality after staining with Trypan blue and of the cell count. All compounds were diluted in RPMI, the final pH of the solution was approximately 7-7.3.

### Example 4

Enzymatic determinations of the activity of glutathione peroxidase (E.C.1.11.1.9.) and glutathione S-transferase (E.C.2.5.1.18.) in hemolyzed human blood in the presence of GSH and of its derivative GSH-C4 were carried out according to the methods of Beutler (Beutler, E., 1984, Red Cell Metabolism. A Manual of Biochemical Methods, 3rd edn. Grune & Stratton, New York). GSH and GSH-C4 were then oxidized by incubation at ambient temperature in the presence of 35% (v/v) H₂O₂ to respectively obtain GSSG and C4-GSSG-C4.

### Example 5

The entry of GSH and GSH-C4 into erythrocytes (RBCs) at 37°C in microcentrifugal test-tubes was measured using the oil-stop method. Erythrocyte suspensions with a 10% hematocrit containing 5 mM of GSH or GSH-C4 and 10 mM GSH were incubated for 2h at 37°C. At different times (0, 5, 15, 30, 60 and 120 min), rates of 600 µl were stratified on 600 µl of bromododecane, centrifuged for 5 min at 10,000 rpm and the GSH and GSH-C4 content measured in RBCs (Beutler, E., 1984, Red Cell Metabolism. A Manual of Biochemical Methods, 3rd edn. Grune & Stratton, New York).

### Example 6

The stability of GSH-C4 in plasma was measured with the following procedure: GSH-C4 (1 mM) was incubated in human plasma at 37°C and for different incubation times (0, 15, 30, 60 and 120 min), rates of 200 µl were ultrafiltered using Amicon Centricon microconcentrators by centrifugation at 2,000 rpm per 30 min. The filtered solution was then analyzed by high performance capillary electrophoresis (HPCE) to measure the content of GSH-C4 and C4-GSSG-C4.

### Example 7

To measure the behavior of GSH-C4 derivatives in infected cells, Madin Darby canine kidney cells (MDCK) were grown in RPMI 1640 to which 5% of heat-decomplemented bovine fetal serum (Flow Laboratories, Italy) was added.

The Senday virus (SV) belonging to the Paramyxovirus family is a virus with non-segmented single stranded RNA of negative polarity. This virus was reproduced by inoculation in the allantoic liquid of embryonated chicken eggs. The monolayers of MDCK cells were infected with SV [3 hemagglutinating units (HAU) x 10⁵ cells]. After incubation for 1 hour at 37°C (adsorption period), the unadsorbed viruses were removed, the monolayers were washed and then incubated in a complete medium containing 2% of bovine fetal serum. The production of viruses by infected cells was determined in the cellular supernatants at different intervals of time from infection (p.i.), measuring the hemagglutinating activity towards type 0 Rh+ human erythrocytes (HAU), according to standard procedures. To measure the antiviral activity, the compounds GSH-C2, GSH-C4, GSH-C6 were diluted in RPMI pH 7.3 and added at the desired concentration, immediately after the adsorption period of the virus. Monkey kidney cells (VERO) were grown in RPMI, to which 5% of serum was added. The human herpes simplex virus type 1 (HSV-1), clinically isolated (TV1), was grown and titered in the Vero cells as described in Palamara, A. T., Perno, C. F., Ciriolo, M. R., Dini, L., Balestra, E., D'Agostini, C. et al. (1995). Evidence for antiviral activity of glutathione: in vitro inhibition of herpes simplex virus type 1 replication. Antiviral Research 27, 237-253.

To obtain the viral infection, monolayers of Vero cells were infected with HSV-1 at a MOI (multiplicity of infection) of 0.03 PFU/cell. After incubation for 1 hour at 37°C (adsorption period) the unadsorbed viruses were removed, the monolayers were washed and then incubated with culture medium containing 2% bovine fetal serum. GSH-C4 was added in identical doses to the ones indicated above immediately after the adsorption period and was maintained in the culture medium until completion of the experiments. Supernatants from infected cells were collected at different times after contact with the virus and tested for the ability to form plaques in Vero cells, using a standard titration method. Similar experiments were also conducted on HSV-1 TK-D305.

The results obtained on the basis of the examples described are summarized below.

### Toxicity of GSH derivatives

It was found that GSH-C4 and GSH-C2 derivatives do not induce any toxic effect or change in cell morphology at the concentrations used in the experiments conducted. On the contrary, the addition of C8 (n-octanoyl) and C12 (n-dodecanoyl) derivatives caused marked toxic effects and damaged the cells of the monolayers of uninfected MDCK. The increase in these effects are dependent on the dose administered and were observed starting from concentrations of 0.1 mM. For this reason their activity on viral replication was not taken into account. Treatment of MDCK cells with Glutathione-C6 caused damages on the uninfected monocellular layers exclusively at the concentration of 10 mM. Some modifications to cellular morphology were found 24 hours after the addition of 2.5 and 5 mM. These substances cause inhibition of viral replication varying from 50% (2.5 mM) to 100% (10 mM) . Due to the limited difference between the cytotoxic doses and the antiviral doses, the GSH-C6 derivative was discarded as it was not optimal.

An inventive selection was then made between the various possible glutathione derivates in order to find which ones could obtain the best effects as antiviral agents and it was surprisingly found that only with GSH-C4 derivatives according to formula I is it possible to obtain adequate antiviral efficacy and simultaneously solve the aforesaid problems correlated to the use of GSH.

### Metabolism of the GSH-C4 derivative

The enzymatic activities of glutathione peroxydase and glutathione S-transferase were measured in hemolyzed human blood in the presence of GSH-C4 or GSH 2 mM as substrates. The activities of glutathione peroxydase and of glutathione S-transferase in the presence of GSH-C4 were 1.8% and 3.5% respectively, compared to those in the presence of GSH. Moreover, GSH and GSH-C4 were chemically oxidized in the corresponding disulphide forms (GSSG and C4-GSSG-C4). The activity of glutathione reductase was measured on both substrates and the results obtained showed that the oxidized form of C4-GSSG-C4 is gradually reduced with an enzymatic activity of 0.63 IU/g of hemoglobin (Hb), a Km of 50 mM and a Vmax equal to 12.6 µmol/min/mg Hb, while the oxidized form of GSH is reduced by glutathione reductase with an activity of 3.0 IU/g Hb, a Km 1.3 mM and a Vmax 6.3 µmol/min/mg Hb. These results show that once the oxidized dimer of GSH-C4 has formed, the cellular glutathione reductase is unable to efficiently reduce the compound.

### Entry of GSH-C4 in the erythrocytes

Entry of GSH-C4 in the erythrocytes was measured and compared with GSH entry. The results obtained show that GSH-C4 crosses the membranes of the erythrocytes more rapidly than GSH, in this way solving the technical problem addressed by the present invention; in fact, the entry speed (calculated in the first 30 minutes) was 4.33 nmol/min/ml RBCs for GSH-C4 and 2.33 nmol/min/ml RBCs for GSH.

### Effects of GSH-C4 on Sendai virus replication.

It was observed that the addition of GSH-C2 to MDCK cells infected with the Sendai virus caused a slight reduction in viral replication measured as hemagglutinating activity in the supernatant.

In particular, addition of the glutathione derivative at concentrations of 5.0 and 7.5 mM causes inhibition of the viral titer of 30% and 40% respectively.

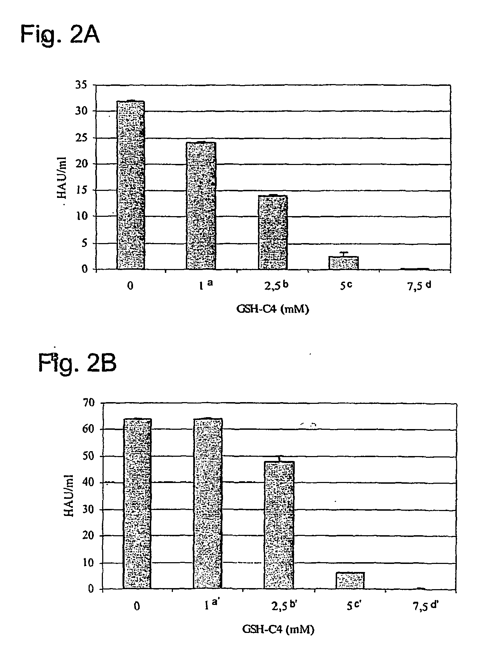

Vice versa, the addition of GSH-C4 showed a much greater effect. The effect of GSH-C4 on reproduction of the Senday virus in MDCK cells is also shown in Figure 2.

MDCK cells were infected for 1 hour with 3 hemagglutinating units (HAU) x 10⁵ cells. The cells were washed and then cultivated in the presence of different concentrations of GSH-C4 (range 0-7.5 mM) for 2 days. Production of the virus was assayed at 24 hours (panel A) and 48 hours (panel B) measuring the hemagglutinating activity on type 0 Rh+ human erythrocytes. Inhibition of Sendai virus replication is dependent on the dose of GSH-C4 administered. A degree of inhibition varying from 88% (24h p.i.) to 93% (48h p.i.) was obtained in the presence of 5 mM of GSH-C4. In the supernatant of treated cells with composition containing GSH-C4 7.5 mM no viruses were found. The dose of 7.5 mM of GSH-C4 therefore proved to be optimal as it is sufficient to produce an excellent antiviral effect, without being toxic for the cells, as also confirmed by microscopic examination of the monolayers. 50% of inhibition of viral reproduction at 48h (EC50) is obtained with GSH-C4 at a dose of only 3.6 mM, while a total of 7.6 mM of GSH are required to obtain the same result.

### Effect of GSH-C4 on HSV-1 replication

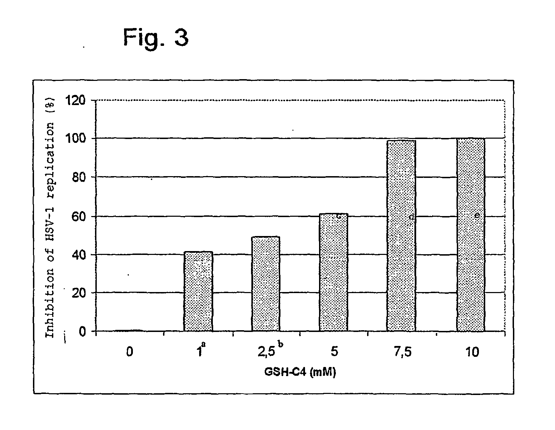

The effect of different doses of GSH-C4 on HSV-1 replication in VERO cells is shown in Figure 3, which indicates the percentages of inhibition of replication of the virus 48h after infection.

Vero cells were infected for 1 hour with HSV-1 at a MOI of 0.03 PFU/cell. After repeated washings, GSH-C4 was added at different concentrations (range 0-10 mM) to the cell cultures. Replication of the viruses was tested in the Vero cells 48h after infection using the plaque technique. Figure 3 shows the results of four different experiments which are concordant within 10% of the values indicated.

The results obtained show that inhibition of virus replication is dependent on the dose of GSH-C4 administered. A marked decrease (60% of inhibition compared to the control) in viral replication was obtained by adding 7.5 mM of GSH-C4. At the dose of 10 mM, no virus particles were detected in the cellular supernatant. The same inhibition was found 72h after infection.

The addition of GSH at a concentration of 10 mM does not induce complete inhibition of the virus and only produces a reduction in virus replication of around 2.5 log. Moreover, GSH-C4 protects the Vero cells from the cytopathic effects induced by the virus and does not induce any toxic effects in uninfected Vero cells. The effect of GSH-C4 was also assessed on a strain of defective virus for thymidine kinase (Δ305).
Vero cells were infected for 1h at 37°C with the strain Δ305 and then kept in culture in the presence of different concentrations of GSH-C4. The production of viruses was measured 24, 48 and 72h after infection by assay of the plaque forming units.

The table shows the results of one experiment representative of three. The variability between results obtained in the various experiments did not exceed 10%.

**Tab. 1 Effects of GSH-C4 on HSV-1 TK-**

| | HSV-1 TK- (pfu/ml) | | |
|---|---|---|---|
| | 24h | 48h | 72h |
| Ctr. | 1.38 x 10⁵ | 1.96 x 10⁵ | 2.56 x 10⁶ |
| 5 mM | 2.59 x 10⁵ | 2.42 x 10⁶ | 2.13 x 10⁶ |
| 7.5 mM | 1.59 x 10⁵ | 2.56 x 10⁵ | 4.33 x 10⁵ |
| 10 mM | 1.53 X 10⁵ | 2.6 X 10⁵ | 3.44 X 10⁵ |

The results obtained show that GSH-C4 is less active against the strain TK⁻ compared with when it is used on the wild type strain. In fact a significant inhibition of virus titer (approximately 1 log) is observed only at a concentration of 7.5 mM 72h after infection.

Finally, it is apparent that as it has been proven experimentally that the derivatives according to the present invention are efficacious in the treatment of diseases deriving from the Sendai virus and therefore of paramyxoviruses, they are also efficacious against orthomyxoviruses. Moreover, as all the tests performed are concordant in confirming efficacy against various viruses, it is obvious to infer that GSH-C4 derivatives of formula I are antiviral agents.

Finally, although the examples are relative to the GSH-C4 derivative in which the only SH group is not substituted, it is legitimate to assume that all the derivatives in which the SH group of GSH-C4 according to the present invention is substituted with protection groups are equally efficacious in antiviral treatments.

## Claims

1. Glutathione derivatives of formula I: 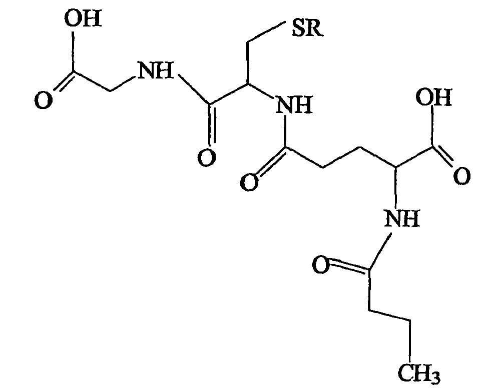 wherein R is a thiol protecting group.

2. Glutathione derivatives of formula I, wherein R is H, or acetyl.

3. Derivatives as claimed in claim 1 or 2, for use as medicament.

4. Derivatives as claimed in claim 1 or 2, for use as antiviral medicament.

5. Derivatives as claimed in claim 1 or 2, for the treatment of diseases caused by Paramyxoviruses.

6. Derivatives as claimed in claim 1 or 2, for the treatment of diseases caused by Orthomyxoviruses.

7. Derivatives as claimed in claim 1, for use as medicament for the treatment of diseases caused by Herpes Simplex-1.

8. Derivatives as claimed in claim 1, for use as medicament for the treatment of diseases caused by HIV.

9. Pharmaceutical preparation **characterized in that** it comprises a glutathione derivative having the general formula (I) as claimed in claim 1 or 2 or a pharmaceutically acceptable salt thereof and at least a pharmaceutically acceptable excipient and/or diluent.

10. Use of a glutathione derivative of formula (I) as claimed in claim 1 or 2 for the production of an antiviral pharmaceutical preparation.

11. Use of a glutathione derivative of formula (I) as claimed in claim 1 or 2 for the production of a pharmaceutical preparation for the treatment of diseases caused by Paramyxoviruses.

12. Use of a glutathione derivative of formula (I) as claimed in claim 1 or 2 for the production of a pharmaceutical preparation for the treatment of diseases caused by Orthomyxoviruses.

13. Use of a glutathione derivative of formula (I) as claimed in claim 1 or 2 for the production of a pharmaceutical preparation for the treatment of diseases caused by Herpes simplex-1.

14. Use of a glutathione derivative of formula (I) as claimed in claim 1 or 2 for the production of a pharmaceutical preparation for the treatment of diseases caused by HIV.

## Patentansprüche

1. Glutathionderivate der Formel I: 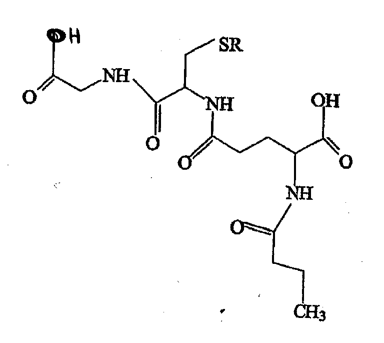 wobei R eine Thiolschutzgruppe ist.

2. Gluthationderivate nach Formel I, wobei R H oder Acetyl ist.

3. Derivate nach Anspruch 1 oder 2 zur Verwendung als Medikament.

4. Derivate nach Anspruch 1 oder 2 zur Verwendung als antivirales Medikament.

5. Derivate nach Anspruch 1 oder 2 zur Behandlung von durch Paramyxoviren verursachten Erkrankungen.

6. Derivate nach Anspruch 1 oder 2 zur Behandlung von durch Orthomyxoviren verursachten Erkrankungen.

7. Derivate nach Anspruch 1 zur Verwendung als Medikament zur Behandlung von durch Herpes simplex-1 verursachten Erkrankungen.

8. Derivate nach Anspruch 1 zur Verwendung als Medikament zur Behandlung von durch HIV verursachten Erkrankungen.

9. Pharmazeutische Zubereitung, **dadurch gekennzeichnet, dass** sie ein Glutathionderivat der allgemeinen Formel (I) nach Anspruch 1 oder 2 oder ein pharmazeutisch verträgliches Salz hiervon und zumindest einen pharmazeutisch verträglichen Hilfsstoff und/oder ein pharmazeutisch verträgliches Streckmittel umfasst.

10. Verwendung eines Glutathionderivats der Formel (I) nach Anspruch 1 oder 2 zur Herstellung einer antiviralen pharmazeutischen Zusammensetzung.

11. Verwendung eines Glutathionderivats der Formel (I) nach Anspruch 1 oder 2 zur Herstellung einer pharmazeutischen Zusammensetzung zur Behandlung von durch Paramyxoviren verursachten Erkrankungen.

12. Verwendung eines Glutathionderivats der Formel (I) nach Anspruch 1 oder 2 zur Herstellung einer pharmazeutischen Zusammensetzung zur Behandlung von durch Orthomyxoviren verursachten Erkrankungen.

13. Verwendung eines Glutathionderivats der Formel (I) nach Anspruch 1 oder 2 zur Herstellung einer pharmazeutischen Zusammensetzung zur Behandlung von durch Herpes simplex-1 verursachten Erkrankungen.

14. Verwendung eines Glutathionderivats der Formel (I) nach Anspruch 1 oder 2 zur Herstellung einer pharmazeutischen Zusammensetzung zur Behandlung von durch HIV verursachten Erkrankungen.

## Revendications

1. Dérivés du glutathion de formule I : 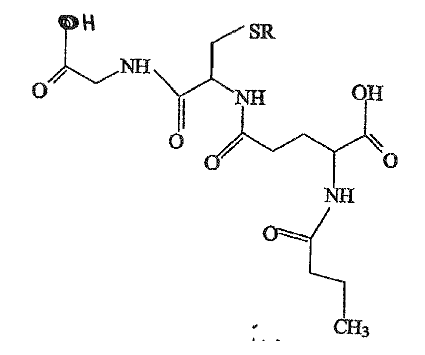 dans lesquels R est un groupe thiol protecteur.

2. Dérivés du glutathion de formule I, dans lesquels R est H, ou acétyl.

3. Dérivés tels que revendiqués dans la revendication 1 ou 2, destinés à être utilisés comme médicament.

4. Dérivés tels que revendiqués dans la revendication 1 ou 2, destinés à être utilisés comme médicament antiviral.

5. Dérivés tels que revendiqués dans la revendication 1 ou 2, pour le traitement de maladies causées par les Paramyxovirus.

6. Dérivés tels que revendiqués dans la revendication 1 ou 2, pour le traitement de maladies causées par les Orthomyxovirus.

7. Dérivés tels que revendiqués dans la revendication 1, destinés à être utilisés comme médicament pour le traitement de maladies causées par Herpès Simplex-1.

8. Dérivés tels que revendiqués dans la revendication 1, destinés à être utilisés comme médicament pour le traitement de maladies causées par HIV.

9. Préparation pharmaceutique **caractérisée en ce qu'**elle comprend un dérivé du glutathion ayant la formule générale (I) telle que revendiquée dans la revendication 1 ou 2 ou un sel pharmaceutiquement acceptable de celui-ci et au moins un excipient et / ou diluent pharmaceutiquement acceptable.

10. Utilisation d'un dérivé du glutathion de formule (I) tel que revendiqué dans la revendication 1 ou 2 pour la production d'une préparation pharmaceutique antivirale.

11. Utilisation d'un dérivé du glutathion de formule (I) tel que revendiqué dans la revendication 1 ou 2 pour la production d'une préparation pharmaceutique pour le traitement de maladies causées par les Paramyxovirus.

12. Utilisation d'un dérivé du glutathion de formule (I) tel que revendiqué dans la revendication 1 ou 2 pour la production d'une préparation pharmaceutique pour le traitement de maladies causées par les Orthomyxovirus.

13. Utilisation d'un dérivé du glutathion de formule (I) tel que revendiqué dans la revendication 1 ou 2 pour la production d'une préparation pharmaceutique pour le traitement de maladies causées par Herpès Simplex-1.

14. Utilisation d'un dérivé du glutathion de formule (I) tel que revendiqué dans la revendication 1 ou 2 pour la production d'une préparation pharmaceutique pour le traitement de maladies causées par HIV.
